# DEMANDE DE BREVET EUROPEEN

(11) **EP 2 628 522 A1**
(43) Date de publication de la demande: **21.08.2013**
(21) Numéro de dépôt: 12155271.5
(22) Date de dépôt: 14.02.2012
(51) Int. Cl.: B01D 53/053, A61M 16/10, F17C 5/00, F24F 3/12, F17D 1/04

(54) **Installation de production sur site de gaz médical à plusieurs capacités en série et ligne de purge**

(71) Demandeur: Air Liquide Medical G.m.b.H., 40235 Düsseldorf (DE)
(72) Inventeur: Maamar, Kais, 45128 Essen (DE); Bongers, Karsten, 47800 Krefeld (DE); Franken, Hartmut, 47829 Krefeld (DE); Neu, Peter, 47249 Duisburg (DE); Sommier, Vincent, 75014 Paris (FR)
(74) Mandataire: Pittis, Olivier

(57) **Abrégé**

L'invention porte sur une installation de production (100) sur site de gaz médical, en particulier d'air de qualité médicale, comprenant une unité de purification (50) de gaz, une unité de compression de gaz (31) alimentant l'unité de purification de gaz (50) avec un gaz à purifier comprimé à une pression supérieure à 1 bar absolu, une ligne principale de gaz (10) reliant fluidiquement l'unité de purification de gaz (50) à au moins un site utilisateur (30), une première capacité (A) de stockage de gaz purifié agencée sur la ligne principale de gaz (10). La ligne principale de gaz (10) comprend en outre une deuxième capacité (B) de stockage de gaz purifié agencée en série en aval de la première capacité (A) de stockage. Une ligne d'échappement (11) à l'atmosphère (en 12) est raccordée fluidiquement à la ligne principale de gaz (10).

## Description

L'invention concerne une installation de production sur site d'air médical, c'est-à-dire au sein d'un bâtiment hospitalier ou analogue, mettant en oeuvre plusieurs capacités de stockage de gaz agencées en série sur la canalisation principale et une ligne de purge connectée à la canalisation principale, notamment via une électrovanne à 3-voies pilotée.

L'air médical utilisé dans les hôpitaux, les cliniques, les centres de soins, les unités d'urgence ou de secours, ou analogues pour la respiration des patients est un médicament dont la composition est donnée par la pharmacopée européenne.

Plus précisément, l'air médical est de l'air ambiant comprimé à une pression supérieure à la pression atmosphérique, typiquement à plusieurs bar, voire à plusieurs dizaines ou même centaines de bar, et contenant (en volume) de 20,4% à 21,4% d'oxygène, au maximum 500 ppm de CO₂, au maximum 5 ppm de CO, au maximum 1 ppm de SO₂, au maximum 2 ppm de NO et de NO₂, au maximum 67 ppm d'eau et au maximum 0,1 mg/m³ d'huile; les vapeurs d'huile éventuellement présentes provenant essentiellement de la compression de l'air.

A noter que les composés susmentionnés autres que l'oxygène (COx, NOx, eau, huile...), sont en fait des impuretés dont la présence est tolérée dans les limites de la pharmacopée mais qui idéalement n'y sont pas présentes.

L'air médical contient en outre de l'azote et peut aussi contenir d'autres composés, tel l'argon.

Actuellement, l'air médical est fourni aux hôpitaux ou analogues sous trois formes, à savoir selon le cas :
- une livraison directe sous forme d'air comprimé, par exemple à une pression absolue de 200 à 300 bar, dans des cylindres, c'est-à-dire des bouteilles ou bonbonnes de gaz, ou des cadres regroupant plusieurs bouteilles ;
- une production sur site par mélange d'oxygène et d'azote de manière à créer des mélanges azote/oxygène, et
- une production directement sur site à partir d'air ambiant traité notamment par compresseurs et chaines de filtration/purification.

Parmi celles-ci, la production d'air directement sur site, par compresseurs et chaines de filtration est la solution la plus répandue. Un tel procédé est par exemple décrit dans le document EP-A-864818.

L'air ambiant est aspiré, comprimé par des compresseurs à une plage de pression allant de 1 bar à 80 bar relatifs. Cet air comprimé est ensuite filtré, c'est-à-dire purifié, via une ou plusieurs étapes de traitement, par exemple par un jeu de filtres et/ou par mise en oeuvre d'un procédé à pression modulée ou PSA (pour *Pressure Swing Adsorption*).

L'air médical ainsi produit peut être stocké dans une ou plusieurs capacités-tampons intermédiaires, puis envoyé dans le réseau de canalisations qui parcourt le bâtiment hospitalier pour approvisionner les salles de soins, les chambres ou autres en air médical. Il est bien évidemment possible, voire indispensable dans certains cas, de procéder à une détende intermédiaire du gaz, par exemple pour passer d'une pression de 10 bar environ dans la capacité de stockage à une pression de 5 ou 8 bars au sein du réseau.

On palie en général à toute rupture d'approvisionnement en air médical, grâce à de l'air médical issu d'une source de réserve ou de secours ou l'air est conservé sous forme gazeuse.

Les autres gaz médicaux utilisés dans les hôpitaux ou les centres de soins, tel l'oxygène, sont aussi fournis de manière analogue à l'air. Les compositions de ces autres gaz sont également données par la pharmacopée européenne.

Ainsi, l'oxygène peut être également produit sur site par procédé PSA en utilisant des adsorbants spécifiques, telles des zéolites X échangées au lithium, permettant de retenir l'azote contenu dans l'air et de produire ainsi de l'oxygène gazeux ayant une pureté typiquement supérieure à 90%, voire à 93% en volume, comme connu du document EP-A-297542.

Toutefois, les procédés de production d'air médical ou d'autres gaz médicaux utilisés sur site (encore appelés procédés *on site*) présentent certains inconvénients.

Tout d'abord, ces procédés ne permettent pas un contrôle aisé de la fiabilité du processus de fabrication.

Ainsi, lorsqu'une unité de production sur site d'air médical fonctionne de façon autonome, le processus de fabrication n'est pas suivi en continu et les interventions sur l'installation se font sur une base d'une planification, c'est-à-dire une maintenance préventive, ou lorsqu'une erreur ou un problème survient sur l'installation, c'est-à-dire une maintenance curative.

Ces interventions sont donc réalisées indépendamment de l'état de l'installation et de sa fiabilité, ce qui n'est pas optimal car elles sont réalisées ou trop tôt, donc sans nécessité réelle, ou trop tard, donc avec une incidence sur le processus de production et éventuellement sur le produit final.

Ensuite, on assiste à des blocages de polluants dans la canalisation principale lorsque l'installation n'est pas conforme. En fait, dans les installations existantes, l'électrovanne de contrôle est une électrovanne dite « à 2 voies » qui est agencée sur la ligne principale.

Si, elle permet d'arrêter une éventuelle pollution en amont de la vanne, cette pollution reste cependant bloquée dans la ligne principale et nécessite une purge totale du système en amont de la vanne. Ceci n'est pas idéal car il nécessite un arrêt de la production de gaz et une intervention manuelle.

Par ailleurs, en cas de courtes ruptures de fourniture d'air dues par exemple à une brève contamination à l'entrée, la source de secours est directement sollicitée. Or, cela pose problème car le volume de secours est limité et donc si la fréquence des ruptures de fourniture est importante, alors on risque d'assécher la source de secours. En d'autres termes, il serait très bénéfique de pouvoir éviter cet inconvénient en réduisant les sollicitations de la source de secours de manière à augmenter son autonomie au fil du temps.

Enfin, l'air produit par les procédés et installations actuels, n'est en général ni analysé, ni validé, ce qui peut poser d'évidents problèmes de conformité et de qualité. D'ailleurs, lorsqu'il est analysé, en cas de « non-conformité », on assiste habituellement soit à une interruption immédiate de la production et passage sur une source d'air de secours, ce qui peut avoir pour conséquence une utilisation abusive de l'air de secours susceptible de conduire à une éventuelle rupture totale de la fourniture en air ; soit à une continuité de fourniture d'un air non conforme et déclenchement en parallèle d'une alerte pour avertir l'utilisateur qui doit alors intervenir de façon manuelle. On comprend que ces solutions ne sont pas non plus idéales.

En définitive, il n'existe actuellement aucune méthode de validation de l'air produit sur site permettant de garantir que l'air produit est bien conforme aux spécifications requises et qui permette d'assurer une fourniture en air médicale efficace et fiable.

En d'autres termes, le problème qui se pose est de proposer une installation de production sur site et en continu d'un gaz médicament, en particulier d'air médical, conformément aux bonnes pratiques de fabrication ou GMP (pour *Good Manufacturing Practice*) et qui permette notamment :
- une supervision de la fiabilité du processus de fabrication avec détection rapide de toute anomalie,
- un contrôle des différentes étapes de production et en particulier de la production finale, avec pour chaque étape, une possibilité de purge permettant ainsi d'arrêter toute contamination ou non-conformité du gaz produit, et/ou
- de réduire l'utilisation des sources de secours à un niveau minimal.

La solution de l'invention est une installation de production sur site de gaz médical, notamment d'air de qualité médicale, comprenant : une unité de purification de gaz, une unité de compression de gaz alimentant l'unité de purification de gaz avec un gaz à purifier comprimé, une ligne principale de gaz reliant fluidiquement l'unité de purification de gaz à au moins un site utilisateur, une première capacité de stockage de gaz purifié agencée sur la ligne principale de gaz, **caractérisée en ce que** la ligne principale de gaz comprend en outre une deuxième capacité de stockage de gaz purifié agencée en série en aval de la première capacité de stockage, et une ligne d'échappement à l'atmosphère est raccordée fluidiquement à la ligne principale de gaz.

Selon le cas, l'installation de l'invention peut comprendre l'une ou plusieurs des caractéristiques techniques suivantes :
- l'unité de compression de gaz alimente l'unité de purification de gaz avec un gaz à purifier comprimé à une pression supérieure à 1 bar absolu.
- l'unité de compression de gaz alimente l'unité de purification de gaz avec de l'air ambiant comprimé.
- l'unité de compression de gaz comprend au moins un compresseur à vis, à pistons, à spirales ou à membranes.
- l'unité de compression de gaz comprend plusieurs compresseurs, notamment agencés en parallèle.
- l'unité de purification de gaz comprend au moins un adsorbeur contenant chacun au moins un lit d'au moins un matériau adsorbant, de préférence au moins 2 adsorbeurs agencés en parallèles et fonctionnant en cycle de type PSA.
- l'unité de purification de gaz comprend en outre un ou plusieurs filtres.
- l'unité de purification de gaz, en particulier les adsorbeurs, permet d'éliminer tout ou partie des impuretés présentes dans l'air ambiant à purifier ou qui y ont été introduites lors de la compression, en particulier la vapeur d'eau, les vapeurs d'huile, les SOx, COx et/ou les NOx, de manière à produire un gaz médical conforme à la pharmacopée, en particulier de l'air médical conforme à la pharmacopée européenne.
- la ligne principale de gaz comprend en outre une électrovanne à 3 voies dont l'une des voies est raccordée fluidiquement à la ligne d'échappement et dont les deux autres voies sont connectées fluidiquement à la ligne principale.
- la vanne est une électrovanne commandée par une unité de pilotage, tel un automate, de préférence l'unité de pilotage reliée électriquement à l'électrovanne à trois voies.
- l'unité de compression de gaz comprend une (ou plusieurs) entrée de gaz alimentée en air atmosphérique.
- la ligne principale relie la première capacité de stockage de gaz purifié à au moins un site utilisateur de gaz, de préférence à un réseau de canalisations au sein d'un bâtiment hospitalier.
- la ligne principale de gaz comporte une deuxième capacité de stockage de gaz purifié située entre la première capacité de stockage et ledit au moins un site utilisateur de gaz. Les première et deuxième capacités sont donc agencées en série sur la ligne principale.
- la ligne principale de gaz se ramifie en aval de la première capacité de stockage en une ligne secondaire connectée fluidiquement en amont à ladite ligne principale et en aval à au moins un site utilisateur de gaz, c'est-à-dire directement ou indirectement par exemple en venant se raccorder à la ligne principale de gaz, ladite ligne secondaire comprenant une troisième capacité de stockage de gaz purifié. Les première et troisième capacités sont donc elles aussi agencées en série, alors que les deuxième et troisième capacités sont elles agencées en parallèles sur la ligne principale et la ligne secondaire respectivement.
- elle comporte en outre une ligne de secours reliant fluidiquement une source de secours, c'est-à-dire une source d'air médical, audit au moins un site utilisateur de gaz, et ce, directement ou indirectement en venant se raccorder à la ligne principale de gaz ou à ladite ligne secondaire.
- elle comporte en outre des électrovannes à au moins 2 voies, typiquement des électrovannes à 2 voies, agencées sur la ligne principale et la ligne secondaire, avec une première électrovanne agencée entre la première capacité et la deuxième capacité de stockage de gaz purifié, et une deuxième électrovanne agencée entre la première capacité et la troisième capacité de stockage de gaz purifié.
- elle comporte en outre des électrovannes à au moins deux voies, agencées sur la ligne principale et la ligne secondaire, avec une troisième électrovanne agencée en aval de la deuxième capacité et/ou une quatrième électrovanne agencée en aval de la troisième capacité.
- elle comporte en outre une première ligne de purge raccordée fluidiquement, en amont, à la ligne principale et, en aval, à la ligne d'échappement, de préférence la première ligne de purge est raccordée fluidiquement à la ligne principale en aval de la deuxième capacité.
- elle comporte en outre une deuxième ligne de purge raccordée fluidiquement, en amont, à la ligne secondaire et, en aval, à la ligne d'échappement, de préférence la deuxième ligne de purge est raccordée fluidiquement à la ligne secondaire en aval de la troisième capacité.
- la première ligne de purge comprend une cinquième électrovanne et/ou la deuxième ligne de purge comprend une sixième électrovanne.
- elle comporte au moins un premier dispositif d'analyse de gaz dont la première ligne de mesure est raccordée fluidiquement à la ligne principale, en amont de l'électrovanne à trois voies.
- elle comporte un deuxième dispositif d'analyse de gaz dont au moins une deuxième ligne de mesure est raccordée fluidiquement à la ligne principale et/ou à la ligne secondaire.
- la deuxième ligne de mesure comprend une septième et/ou une huitième électrovanne.
- l'unité de pilotage commande en outre l'unité de purification de gaz, l'unité de compression de gaz, l'une ou plusieurs des électrovannes et le ou les dispositifs d'analyse de gaz.
- elle comporte un ou plusieurs clapets anti-retour agencés sur tout ou partie des canalisations ou lignes véhiculant le gaz.
- l'électrovanne à 3 voies est agencée en amont de la première capacité de stockage de gaz purifié.

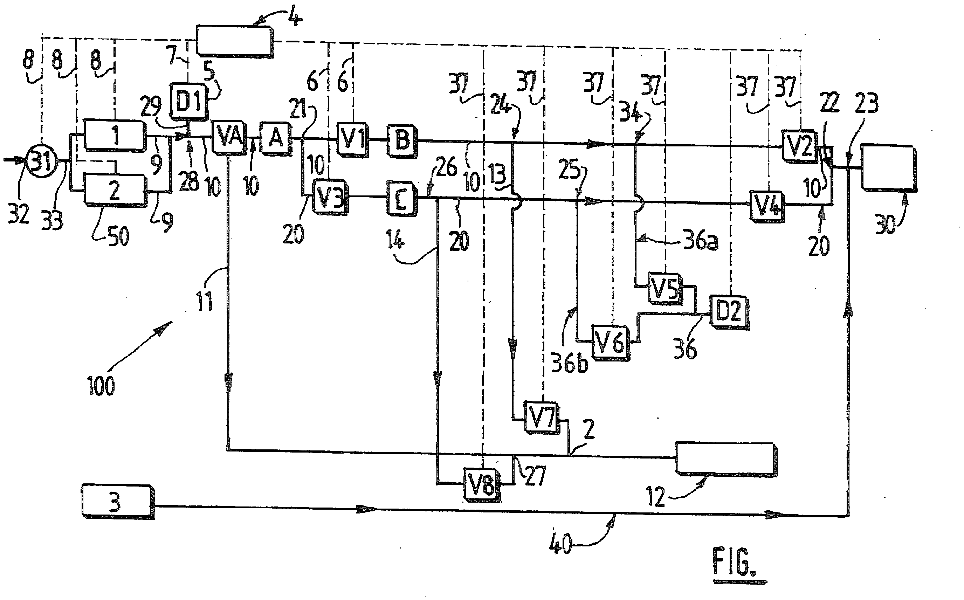

La présente invention va maintenant être décrite plus en détail en références à la Figure annexée qui représente le schéma de principe d'un mode de réalisation d'une installation 100 de production sur site de gaz médicaux selon l'invention, laquelle est raccordée au réseau de canalisations 30 d'un bâtiment hospitalier ou analogue.

Le gaz produit ici est de l'air médical, c'est-à-dire de l'air purifié répondant aux spécifications de la pharmacopée européenne susmentionnée. Toutefois, une telle installation 100 selon l'invention peut être utilisée pour fabriquer d'autres gaz médicaux, par exemple de l'oxygène médical à partir d'air ambiant.

Plus précisément, dans ce mode de réalisation, l'installation de production 100 sur site d'air médical comprend une unité de purification 50 de gaz alimentée par une unité de compression de gaz 31, c'est-à-dire une ou plusieurs compresseurs d'air aspirant de l'air ambiant (à pression atmosphérique : 1 atm) par leur entrée d'alimentation 32 et délivrant de l'air comprimé à une pression supérieure à la pression atmosphérique, par exemple à une pression comprise entre 1 bar et 80 bar absolus. Ce ou ces compresseurs 31 peuvent être un ou des compresseurs à vis, à pistons, à spirales ou à membranes.

L'air comprimé alimente l'unité de purification de gaz 50 qui comprend ici deux adsorbeurs 1, 2 fonctionnant en parallèle selon des cycles de type PSA (Pressure Swing Adsorption), c'est-à-dire que l'un est en phase de production pendant que l'autre est en phase de régénération, et inversement. Typiquement, la durée d'un cycle de production est comprise entre 1 et 30 minutes environ, de préférence de moins de 10 à 15 minutes.

Pour améliorer la purification de l'air et l'élimination des impuretés, on peut également prévoir la présence de filtres, par exemple un filtre à charbon actif et/ou d'autres filtres aptes à stopper tout ou partie des impuretés à éliminer.

Ces adsorbeurs 1, 2 contiennent chacun au moins un lit d'au moins un matériau adsorbant, par exemple des matériaux adsorbants de type zéolites, alumines, charbon actif, gel de silice ou tout autre tamis moléculaire apte à stopper des impuretés présentes dans l'air ambiant.

L'unité de purification de gaz 50 peut aussi comprendre, selon le mode de réalisation considéré, un adsorbeur unique ou plus de 2 adsorbeurs, 1, 2 par exemple au moins 3 adsorbeurs.

Ce type d'adsorbeurs 1, 2 et de cycle PSA ou TSA sont bien connus et, à ce titre, on peut d'ailleurs se reporter par exemple aux documents EP-A-716274, EP-A-718024, EP-A-922482, GB-A-1551348, EP-A-930089.

Dans tous les cas, les adsorbeurs 1, 2 permettent d'éliminer tout ou partie des impuretés éventuellement présentes dans l'air ambiant à purifier ou qui y ont été introduites lors de la compression (en 31), en particulier la vapeur d'eau, les vapeurs d'huile, les SOx, COx et/ou les NOx, de manière à produire un gaz médical conforme à la pharmacopée, en particulier de l'air médical conforme à la pharmacopée européenne.

Ensuite, l'air purifié (ou tout autre gaz médical) produit par l'unité de purification de gaz 50 est récupéré dans des conduites de sortie 9 qui alimentent une ligne principale 10 d'acheminement de gaz, c'est-à-dire une canalisation ou un tuyau d'amenée de gaz, apte à et conçue pour véhiculer l'air purifié ainsi produit d'abord jusqu'à une première capacité A de stockage, c'est-à-dire une capacité-tampon, où l'air médical purifié peut être stocké et homogénéisé, avant d'être envoyé ensuite vers un ou plusieurs sites utilisateurs 30, tel un réseau de canalisations de gaz parcourant un bâtiment hospitalier pour amener l'air médical dans les différentes salles où il doit être utilisé, comme les salles de soins, les salles d'urgence, les salles de réveil, les chambres ou tout autre emplacement.

La ligne principale de gaz 10 relie donc fluidiquement la (ou les) sortie 9 de l'unité de purification de gaz 50 à ladite première capacité A de stockage de manière à l'alimenter en air purifié provenant de l'unité de purification de gaz 50.

Le fonctionnement du ou des compresseurs 31 et les cycles de purification ayant lieu dans l'unité de purification de gaz 50 sont commandés et contrôlés par un dispositif de pilotage 4, par exemple un automate programmable ou analogue, relié à l'unité de purification de gaz 50 par des liaisons électriques 8, tels des câbles électriques.

A noter toutefois que, de façon générale, les communications entre les différents éléments et dispositifs de l'installation, notamment avec l'automate 4, pourraient aussi se faire par liaisons non filaires, par exemple via un ou des dispositifs ou systèmes émetteurs sans fil de type radiofréquence (RF), Bluetooth, Zigbee, wifi, GSM ou GPRS, et une ou des antennes réceptrices permettant d'assurer des transmissions sans fil des données adaptées au type d'émetteur utilisé.

De préférence, l'automate 4 ou similaire est programmé en fonction des besoins du site hospitalier considéré et peut être reprogrammé si les besoins du site change par exemple.

Afin de réguler et contrôler le cheminement du gaz dans la ligne principale de gaz 10, une électrovanne VA est agencée sur ladite ligne principale 10 entre l'unité de purification de gaz 50 et la première capacité A de stockage. L'électrovanne VA est également commandée par le dispositif de pilotage 4 via une liaison électrique 5, tel un câble électrique.

Selon la présente invention, comme expliqué ci-après, une deuxième capacité-tampon B de stockage de gaz purifié est agencée en série sur la ligne principale 10, en aval de la première capacité A de stockage et, par ailleurs, la ligne principale 10 comprend également une ligne d'échappement 11 à l'atmosphère ambiante (en 12) à l'extrémité de laquelle se trouve de préférence un dispositif d'échappement à l'atmosphère, telle une soupape d'échappement (non montré) ou analogue.

La ligne d'échappement 11 à l'atmosphère vient en fait se raccorder fluidiquement à la ligne principale 10 en amont de la première capacité A de stockage.

Ce raccordement se fait via une électrovanne à trois voies VA dont l'une des voies est reliée fluidiquement à ladite ligne d'échappement 11, et dont les deux autres voies sont connectées fluidiquement à la ligne principale 10.

L'air produit par l'unité de purification de gaz 50 traverse donc deux des trois voies de l'électrovanne VA, c'est-à-dire les première et deuxième voie de l'électrovanne VA, lorsqu'elle traverse normalement ladite électrovanne VA, en direction de la capacité tampon A où le gaz purifié peut être stocké.

Par contre, en cas de contamination de la ligne 10 en amont de la vanne VA, cette pollution peut être chassée facilement et efficacement de la portion de conduit contaminée de la ligne principale 10, sans nécessiter une purge totale du système en amont de la vanne VA.

Ceci se fait classiquement en procédant à un balayage de la portion de conduit polluée par des impuretés, avec de l'air pur produit par l'unité de purification de gaz 50. Le flux gazeux entrainant les impuretés est alors évacué via la troisième voie de l'électrovanne VA, vers l'atmosphère par la ligne d'échappement 11 à l'atmosphère ambiante. En d'autres termes, l'air produit par l'unité de purification de gaz 1, 2 va alors entraîner avec lui les polluants et ceux-ci seront rejetés à l'atmosphère (en 12).

L'électrovanne VA à 3 voies permet donc non seulement de bloquer toute pollution éventuelle sur la ligne principale 10 pour la confiner en amont de l'électrovanne VA mais aussi d'évacuer ensuite cette pollution de la ligne principale 10 vers l'extérieur (en 12) et purger ainsi la ligne principale 10 en amont de la vanne VA.

Cette solution permet d'éviter d'arrêter entièrement le processus de production et d'avoir recours au secours 3, c'est-à-dire d'une réserve de gaz pur, en cas de pollution temporaire de l'air aspiré ou créée par la ligne de production.

Par ailleurs, la première capacité-tampon A permet de prendre le relai de livraison du gaz purifié, tel l'air médical, lorsque la vanne VA est en position échappement, c'est-à-dire lorsqu'une purge de la ligne 10 est en cours, de manière à, là encore, réduire la fréquence de recours à la source secours 3.

La première capacité A permet également de protéger l'unité de production 50 des pics de consommation, c'est-à-dire les pics de demande de la part des sites utilisateurs 30, et d'homogénéiser l'air produit par l'unité de production 50.

La surveillance de la composition du gaz produit, tel l'air purifié, délivré par l'unité de production 50 se fait au moyen d'un premier dispositif d'analyse de gaz D1, tel une baie d'analyse ou tout autre analyseur de gaz adapté, dont la ligne de mesure 29 est raccordée fluidiquement (en 28) à la ligne principale 10, en amont de l'électrovanne VA à 3-voies.

Ce dispositif d'analyse de gaz D1 est relié au dispositif de pilotage 4 via une liaison électrique 7, tel un câble électrique ou analogue, de manière à lui transmettre des signaux de mesure et éventuellement autres informations. En fonction des signaux reçus, le dispositif de pilotage 4 peut rétro-agir sur l'électrovanne VA à 3-voies et préférentiellement les autres éléments de l'installation, tel que unité de production 50, compresseur 31..., pour déclencher une purge de la ligne 10 lorsqu'une pollution est détectée.

Plus précisément, pour évaluer la fiabilité du procédé et de l'installation de production, la qualité de l'air produit est analysée via la baie d'analyse D1, notamment les teneurs en H₂O, CO₂ et vapeur d'huile. Des variables de suivi complémentaires (par exemple température, pression, vibrations...) sont par ailleurs collectées à partir de l'installation. Les résultats d'analyse ainsi que les variables de suivi sont par la suite traités par le dispositif de pilotage 4, tel un automate, sur une base statistique ou SPC (pour *Statistical Process Control*) pour définir la fiabilité du procédé de production.

Le traitement des données se fait pour chacune des lignes de production, c'est-à-dire pour chaucun des adsorbeurs 1, 2 de l'unité de production 50 ainsi que les compresseurs 31, sur la base d'éléments classiques de contrôle, tels qu'indicateurs d'aptitude du procédé de production, carte de contrôle des variables, moyenne, limites de contrôle, analyse de tendance des variables...

A partir des résultats et des paramètres prédéfinis, on peut alors déterminer si le procédé de fabrication est fiable ou pas. Si le processus de fabrication n'est plus fiable, la ligne de production concernée, c'est-à-dire l'adsorbeur 1 ou 2 et le compresseur 31 associé, est arrêtée et la deuxième ligne prend le relai pour produire de l'air purifié, alors que l'autre ligne est régénérée, réinitialisée et/ou subit une opération de maintenance.

En cas de non-fiabilité de la seconde ligne, le système bascule alors sur la source de secours 3. En effet, l'installation comprend aussi une ligne de secours 40 reliant fluidiquement une source de secours 3, tel un réservoir de secours contenant de l'air médical, aux sites utilisateur de gaz 30, directement ou indirectement, c'est-à-dire en venant se raccorder en 23 à la ligne principale de gaz 10 ou à une ligne secondaire 20.

La ligne secondaire 20 est en fait une autre ligne de gaz agencée en parallèle de la ligne principale 10 et servant de passage alternatif au gaz venant de la capacité A par exemple et/ou de l'unité de production 50, lorsque la ligne principale 10 est hors usage, par exemple contaminée et/ou subit une opération de maintenance.

Dit autrement, la ligne principale de gaz 10 se ramifie (en 21) en aval de la première capacité A en la ligne secondaire 20. Celle-ci 20 vient donc se connecter fluidiquement, de son côté amont (en 21) à ladite ligne principale 10 et, par son extrémité aval à au moins un site utilisateur de gaz (30), directement ou indirectement, c'est-à-dire en venant se raccorder (en 22) à la ligne principale de gaz 10 par exemple.

Par ailleurs, la ligne 10 comprend également une deuxième capacité-tampon B de stockage de gaz purifié située entre la première capacité A et le ou les sites utilisateur de gaz, tel un réseau de canalisations 30 d'un bâtiment hospitalier, alors que la ligne secondaire 20 comprend, quant à elle, une troisième capacité C de stockage de gaz purifié.

Ces capacités B, C servent à alimenter le ou les sites utilisateurs 30 en gaz respiratoire. En fait, les capacités B et C servent à fournir de l'air médical en alternance, c'est-à-dire que pendant qu'une capacité (B par exemple) est en cours de remplissage ou d'analyse de conformité, la deuxième (C respectivement) délivre le gaz au réseau hospitalier 30, ou inversement.

D'une façon générale, une maintenance de l'installation 100 est déclenchée par l'automate 4 sur une base prévisionnelle lorsque les paramètres de production d'une ou des deux unités de production 1, 2 atteignent un seuil prédéterminé.

On note par ailleurs que des électrovannes à 2-voies sont agencées sur la ligne principale 10 et la ligne secondaire 20. Plus précisément, une première électrovanne V1 est agencée entre la première capacité A et la deuxième capacité B, et une deuxième électrovanne V3 est agencée entre la première capacité A et la troisième capacité C de stockage de gaz purifié. En outre, une troisième électrovanne V2 agencée en aval de la deuxième capacité B et une quatrième électrovanne V4 est agencée en aval de la troisième capacité C.

Ces électrovannes à 2-voies sont commandées par le dispositif de pilotage 4 via des liaisons électriques 6, 37, tels des câbles ou analogues, et permettent de contrôler le passage du gaz dans les lignes 10, 20 et donc à sectionner les canalisations 10, 20 en tronçons bien déterminés, et aussi à gérer et/ou à piloter les entées et/ou sorties de gaz des capacités B et C.

L'installation 100 comprend par ailleurs une première ligne de purge 13 raccordée fluidiquement, par son extrémité amont 24, à la ligne principale 10, de préférence en aval de la deuxième capacité B, et, par son extrémité aval 25, à la ligne d'échappement 11, ainsi qu'une deuxième ligne de purge 14 raccordée fluidiquement, par son extrémité amont 26, à la ligne secondaire 20, de préférence en aval de la troisième capacité C, et, par son extrémité aval 27, à la ligne d'échappement 11.

La première ligne de purge 13 comprend une cinquième électrovanne V7 et la deuxième ligne de purge 14 comprend une sixième électrovanne V8 servant à contrôler le passage du gaz vers la ligne d'échappement 11. Le dispositif de pilotage 4 commande aussi les électrovannes V7, V8 via des liaisons électriques 37.

Ces lignes de purge 13, 14 permettent de purger les portions de lignes 10, 20 ainsi que les capacités B et C, respectivement, situées en amont des électrovannes V2 et V4, respectivement.

Un deuxième dispositif d'analyse de gaz D2, telle une baie d'analyse ou analogue, est prévu et comporte au moins une deuxième ligne de mesure 36, se ramifiant en deux sous-tronçons 36a, 36b, est raccordée fluidiquement (en 34, 35) à la ligne principale 10 et à la ligne secondaire 20, notamment par le biais des sous-tronçons 36a, 36b. De préférence, la deuxième ligne de mesure 36, 36a, 36b comprend une septième V5 et/ou une huitième V6 électrovanne.

Ce deuxième dispositif d'analyse de gaz D2 permet de déterminer la composition du gaz circulant dans les lignes principale 10 et secondaire 20, en aval des capacités B, C, c'est-à-dire qu'il permet d'analyser en discontinu le gaz issu des capacités B et C de manière à pouvoir déterminer que la composition du gaz qui s'y trouve est conforme aux spécifications, c'est-à-dire ne contient plus de polluants en quantité inacceptable par exemple.

Comme précédemment, ce deuxième dispositif d'analyse de gaz D2 coopère avec le dispositif de pilotage 4 qui lui-même commande les électrovannes V5, V6 via des liaisons électriques 37.

L'alimentation électrique de l'installation 100 est réalisée de manière classique par du courant du secteur, par exemple à une tension entre 1 et 600 V, typiquement 24 V, 230 V ou 400 V.

Si besoin est, des moyens de mesure (non montrés) peuvent être prévus pour déterminer la pression de l'air médical contenu dans les capacités A, B, C de stockage et d'homogénéisation du gaz et éventuellement rétro-agir, via le dispositif de pilotage 4, sur l'unité de compression 31 et/ou l'unité de production 50 de sorte à réguler la production du gaz, tel l'air, en prenant en compte la (ou les) pression ainsi mesurées. Par exemple, les moyens de pilotage 4 peuvent être programmés pour commander l'arrêt de la source de débit 31 et/ou le déclenchement d'une alarme sonore et/ou visuelle, lorsqu'un capteur de pression, agencé au niveau de la capacité tampon A, détecte une pression ou une différence de pression supérieure ou, à l'inverse, inférieure à une valeur-seuil préfixée. Ce type de régulation en pression est parfaitement connu et ne sera pas détaillé ici.

En outre, afin d'assurer une purification encore plus efficace de l'air aspiré par le compresseur 31, on peut également prévoir un (ou plusieurs) dispositif de filtration mécanique (non détaillé) agencé en un ou plusieurs sites, entre la source d'air 31 et le réseau hospitalier 30. Par exemple, l'unité de compression peut comprendre un ou des filtres en entrée 32 et/ou en sortie 33 pour retenir les poussières contenues dans l'air ambiant et les condensats dues à la compression, par exemple des filtres ou séparateurs cycloniques, des filtres micronique ou analogues.

De façon générale, l'installation 100 de production sur site d'air médical, c'est-à-dire au sein d'un bâtiment hospitalier ou analogue, de l'invention met donc en oeuvre une électrovanne à trois voies apte à évacuer vers l'atmosphère, du gaz produit contaminé par des impuretés, par l'intermédiaire d'une ligne de purge ou d'échappement 11, reliée à l'une des voies de l'électrovanne VA, en réponse à une détection de ladite contamination par un dispositif d'analyse D1 coopérant avec un dispositif de pilotage 4.

L'installation de production 100 d'air médical de l'invention est utilisable directement sur site d'utilisation du gaz, c'est-à-dire directement au sein d'un bâtiment hospitalier ou analogue. Elle peut donc être installée directement dans une pièce du bâtiment hospitalier ou alors à l'extérieur dudit bâtiment ou dans des conteneurs et reliée au réseau 30 de canalisations acheminant le gaz à l'intérieur de celui-ci.

## Revendications

1. Installation de production (100) sur site de gaz médical comprenant :
- une unité de purification (50) de gaz,
- une unité de compression de gaz (31) alimentant l'unité de purification de gaz (50) avec un gaz à purifier comprimé,
- une ligne principale de gaz (10) reliant fluidiquement l'unité de purification de gaz (50) à au moins un site utilisateur (30),
- une première capacité (A) de stockage de gaz purifié agencée sur la ligne principale de gaz (10),
**caractérisée en ce que** :
- la ligne principale de gaz (10) comprend en outre une deuxième capacité (B) de stockage de gaz purifié agencée en série en aval de la première capacité (A) de stockage, et
- une ligne d'échappement (11) à l'atmosphère (en 12) est raccordée fluidiquement à la ligne principale de gaz (10).

2. Installation selon la revendication précédente, **caractérisée en ce que** l'unité de compression de gaz (31) comprend au moins un compresseur à vis, à pistons, à spirales ou à membrane, de l'unité de compression de gaz (31) comprend une entrée de gaz (32) alimentée en air atmosphérique.

3. Installation selon l'une des revendications précédentes, **caractérisée en ce que** l'unité de purification de gaz (50) comprend au moins un adsorbeur (1, 2) contenant au moins un lit d'au moins un matériau adsorbant.

4. Installation selon l'une des revendications précédentes, **caractérisée en ce que** la ligne principale de gaz (10) comprend en outre une électrovanne (VA) à 3 voies dont l'une des voies est raccordée fluidiquement à la ligne d'échappement (11) et dont les deux autres voies sont connectées fluidiquement à la ligne principale (10).

5. Installation selon l'une des revendications précédentes, **caractérisée en ce que** l' électrovanne (VA) à trois voies est commandée par une unité de pilotage (4).

6. Installation selon l'une des revendications précédentes, **caractérisée en ce qu'**une première électrovanne (V1) à deux voies est agencée sur la ligne principale de gaz (10) entre la première capacité (A) et la deuxième capacité (B) de stockage de gaz purifié, de préférence une troisième électrovanne (V2) est agencée sur la ligne principale de gaz (10) en aval de la deuxième capacité (B).

7. Installation selon l'une des revendications précédentes, **caractérisée en ce que** l'électrovanne (VA) à 3 voies est agencée en amont de la première capacité (A) de stockage de gaz purifié.

8. Installation selon l'une des revendications précédentes, **caractérisée en ce que** la ligne principale de gaz (10) se ramifie (en 21) en aval de la première capacité (A) de stockage en une ligne secondaire (20) connectée fluidiquement en amont (en 21) à ladite ligne principale (10) et en aval à au moins un site utilisateur de gaz (30).

9. Installation selon l'une des revendications précédentes, **caractérisée en ce que** ladite ligne secondaire (20) comprend une troisième capacité (C) de stockage de gaz purifié.

10. Installation selon l'une des revendications précédentes, **caractérisée en ce qu'**elle comporte en outre une ligne de secours (40) reliant fluidiquement une source de secours (3) audit au moins un site utilisateur de gaz (30).

11. Installation selon l'une des revendications précédentes, **caractérisée en ce qu**'une deuxième électrovanne (V3) est agencée sur la ligne secondaire (20) entre la première capacité (A) et la troisième capacité (C) de stockage de gaz purifié, de préférence une quatrième électrovanne (V4) est agencée en aval de la troisième capacité (C) sur la ligne secondaire (20).

12. Installation selon l'une des revendications précédentes, **caractérisée en ce qu'**elle comporte en outre une première ligne de purge (13) raccordée fluidiquement, en amont (24), à la ligne principale (10) et, en aval (25), à la ligne d'échappement (11), et une deuxième ligne de purge (14) raccordée fluidiquement, en amont (26), à la ligne secondaire (20) et, en aval (27), à la ligne d'échappement (11).

13. Installation selon l'une des revendications précédentes, **caractérisée en ce que** la première ligne de purge (13) comprend une cinquième électrovanne (V7) et la deuxième ligne de purge (14) comprend une sixième électrovanne (V8).

14. Installation selon l'une des revendications précédentes, **caractérisée en ce qu**'elle comporte au moins un premier dispositif d'analyse de gaz (D1) dont la première ligne de mesure (29) est raccordée fluidiquement (en 28) à la ligne principale (10), en amont de l'électrovanne à trois voies (VA) et/ou elle comporte un deuxième dispositif d'analyse de gaz (D2) dont au moins une deuxième ligne de mesure (36, 36a, 36b) est raccordée fluidiquement (en 34, 35) à la ligne principale (10) et/ou à la ligne secondaire (20).

15. Installation selon l'une des revendications précédentes, **caractérisée en ce que** l'unité de pilotage (4) commande en outre l'unité de purification (50 ; 1, 2) de gaz, l'unité de compression de gaz (31), l'une ou plusieurs des électrovannes (V1 à V8), et/ou le ou les dispositifs d'analyse (D1, D2).
